# EUROPEAN PATENT APPLICATION

(11) **EP 1 767 631 A1**
(43) Date of publication of application: **28.03.2007**
(21) Application number: 06121200.7
(22) Date of filing: 25.09.2006
(51) Int. Cl.: C12N 15/10

(54) **A method for isolating RNA**

(30) Priority: 26.09.2005 EP 05108876
(71) Applicant: Nexttec GmbH, 51377 Leverkusen (DE)
(72) Inventor: Fritzemeier, Nina, 32816 Schieder (DE); Brem, Gottfried, 86567 Hilgertshausen (DE); Leiser, Robert-Matthias, 42697 Solingen (DE); Balayan, Hamlet, 375005 Yerevan (AM)
(74) Representative: von Kreisler Selting Werner

(57) **Abstract**

A method for isolating RNA from RNA containing samples wherein the RNA containing sample is treated with at least one DNase and/or another enzyme like proteases or collagenases for purification of RNA in presence of a complex of ribonucleosides and the oxovanadyl ion which is capable of inhibiting RNases present in the RNA containing samples and removing the complex of ribonucleosides and the oxovanadyl ion prior to down-stream processing of the RNA of the RNA containing sample by contacting the sample of the forgoing step with a chelating agent immobilized to a support, the chelating agent having sufficient affinity to the complex of ribonucleosides and the oxovanadyl ion so that it binds to the chemical entity and the RNA containing sample is freed from the inhibitor.

## Description

The invention pertains to a method for isolating RNA, a support for performing the method of the invention, a method for manufacturing of the support as well as the use of the support of the invention.

The well known and established RNA isolation methods are mostly based on the usage of high concentrated solutions of chaotropic salts or organic solvents like phenol mixtures. This approach leads i.a. to an immediate inhibition of RNases. However, this approach is not applicable for a one step RNA purification. A one step approach needs the combination of an enzymatic lyses, the protection of RNA from RNases and the separation of RNA (or single stranded DNA) from dsDNA. A widely used RNase inhibitor is the ribonucleoside-vanadyl complex, which forms a transition state complex with RNases. These complexes have a very low dissociation constant (10⁻⁷ times lower than the dissociation constant of the enzyme-substrate complex) and are therefore such powerful RNase inhibitors (Berger in Methods of Enzymology, 152 (1987) 227-236, Academic Press London). In that method, 8-hydroxyquinoline was used as an indicator substance for removal of ribonucleoside-vanadyl complex. The ribosyl-vanadyl complexes have been widely used in the past, because they allow an enzymatic tissue lysis with a very rapid inactivation of RNases and a simultaneous degradation of DNA by utilizing a DNase I-treatment directly in the lysate. A disadvantage is that the vanadyl complexes are very strong inhibitors not only for RNases, but also other nucleic acid modifying enzymes like reverse transcriptases and Taq polymerase. Consequently, the inhibitors have to be carefully removed from the RNA preparation before starting any down-streaming enzymatic reaction like reverse transcription and polymerase chain reaction (PCR). A method for removal of ribosyl-vanadyl complexes used an extraction with phenol mixtures. Since the usage of phenol and other organic solvents due to their harmful properties, has been widely removed from nucleic acid purification protocols and the application of this kind of inhibitors has been omitted as well.

P. Blackburn et al. in "The Journal of Biological Chemistry", Vol. 252. No. 15, pp. 5904-5910 (1977) disclose a soluble ribonuclease inhibitor from the human placenta which has been purified 4000-fold by a combination of ion exchange and affinity chromatography.

An object of the invention was to provide a method which allows for use of inhibitors of the RNases which can be removed under avoidance of phenol extraction.

This goal is achieved by using a method for isolating RNA from RNA containing samples wherein the RNA containing sample is treated with at least one DNase and/or another enzyme like proteases or collagenases for purification of RNA in presence of a complex of ribonucleosides and the oxovanadyl ion which is capable of inhibiting RNases present in the RNA containing samples and removing the complex of ribonucleosides and the oxovanadyl ion prior to down-stream processing of the RNA of the RNA containing sample by contacting the sample of the forgoing step with a chelating agent immobilized to a support, the chelating agent having sufficient affinity to the complex of ribonucleosides and the oxovanadyl ion so that it binds to the chemical entity and the RNA containing sample is freed from the inhibitor.

Preferably the RNA containing sample is a cell, tissue, body fluid, virus particle also in its lysed or otherwise disintegrated state.

The term "chelating agent" is well-known to the skilled person. The chelating agent is a chemical entity comprising at least one structural moiety interacting with the oxovanadyl ion and/or the ribonucleoside-oxovanadyl-complex.

The chelating agent immobilized on the support comprises for example the structural element verified in 8-hydroxyquinoline or its derivatives. Furthermore, ethylendiamintetra-acetat (EDTA), bipyridin, ethylene diamin, phenanthroline, oxalat, tartrat, dimethylglyoxime, diethylentriamin, can be used.

In another embodiment the chelating agent comprises a phosphonic acid moiety or a salt thereof. Furthermore, it may be a phosphonic acid derivative such as an amide or an ester. In still another embodiment also di-, tri-, tetra- or even higher carboxylic acids, their salts or derivatives, such as amides, esters or nitriles can be used.

According to the invention the support is a porous inorganic material selected from the group comprising inorganic metal oxides, such as oxides of aluminium, titanium, zirconium, silicon oxides, iron oxides, controlled pore glass (CPG), diatomaceous earth and combinations thereof.

Subject matter of the invention is also a support comprising an inorganic or organic polymer with immobilized chemical moieties which exhibit an affinity to inhibitors of RNases.

The support of the invention can be manufactured by contacting a reactive chemical having a moiety with affinity to a an inhibitor of RNases to the support or an activated support.

In one embodiment the surface of an inorganic support is coated with a substance obtained by polymerization of monomers having chelating functional groups which are capable to interact with the oxovanadyl and/or ribonucleoside-oxovanadyl-complex. In order to obtain spatially cross-linked polymer layers the polymerization can be performed in presence of bifunctional comonomers or oligomers. For example, the coating can be established by mixing the inorganic support with hydroxylvinylquinolin a bifunctional monomer and, if necessary, a polymerization catalyst. As monomers or bifunctional monomers or copolymers can be used organic molecules having one or two or more ethylenically unsaturated compounds or other functional groups which may be polymerized such as carboxyl groups and amides or acrylates and so on. There is a plethora of different monomers and copolymers which are suitable for coating an inorganic support and readily accessible for the skilled person. It is also possible to use an organic support which is functionalized with the respective chelating agents. If more or less inert organic materials are used methods for coating of these polymers can also be employed. The respective chemical reactions belong to the arsenal of a chemist having expertise in polymer-chemistry either organic or inorganic or both.

The invention is further illustrated by means of the following non-limiting examples.

### Example 1: Coating of the surface of macro-porous silica particles with a polymer containing 8-hydroxyquinolin residues

The method is based on polymerization of vinyl monomers with chelating functional groups onto the surface of an inorganic support. In order to obtain spatially cross-linked polymer layers polymerization has been performed in presence of bifunctional divinyl compounds.
As vinyl compound 8-hydroxyvinylquinolin and as bifunctional divinyl compound divinyl benzene have been used.

To 30 g of macro-porous silica have been added under stirring 6,7 g of 8-hydroxyvinylquinolin, 0,75 g of divinyl benzene and 0,04 g of dinitrilazo-bis-isobutyric acid (as the initiator of polymerisation). Benzene has been used as solvent. The polymerisation has been performed at 70 °C for 7 h. The obtained product has been separated from the reaction mix by filtration, washed sequentially with dimethyl formamid, ethanol and a water-acetone mixture. Finally, the product has been dried in a desiccator.

### Example 2. Grafting of 8-hydroxyquinolin into polymer coatings on the surface of macro-porous silica particles

The method is based on the chemical interaction of reactive functional groups inside a polymeric sorbent or inside a polymer layer on the surface of an inorganic support with monomeric organic compounds with chelating groups.
In the present example macro-porous silica (30 g) with a polymer coat made from styrene-divinyl benzene has been used, whereby the copolymer represented 12 % of the silica mass. The divinyl copolymer represents 7% of the mass of the copolymer. As initiator of polymerisation has been used benzoyl peroxide. The polymerisation took place by 80 °C for 7h.
This support has been treated by nitration, followed by reducing the obtained nitro-co-polymer, diazotization of received polyaminostyrene and azocoupling of the obtained product with 8-hydroxyquinolin (by mass. ratio of co-polymer and 8-HQ 1 : 1) by the well-known methods.

### Example 3: RNA preparation from bacteria (E. coli) using RNase-Inhibitor vanadyl-ribosyl complex (VRC) and chelating sorbent from example 1

### Essential reagents:

**(A) Tris I:** 20 mM Tris HCl pH 7,4, 10mM NaCl, 3mM Magnesiumacetate
**(B) Lysis buffer I:** Tris I + 5% (w/w) Sucrose + 1,2% (w/w) Triton N-101
**(C)VRC** (Sigma-Aldrich 94740): 200 mM

Additional reagents: Lysozyme, Proteinase K

### Preparation of lysis buffer for 10 samples:

720 µl Tris buffer I
240 µL Lysis buffer I
96u L RVC

### Lysis

An overnight culture of E.coli (200 µL) has been centrifuged in Eppendorf tubes and the supernatant discarded.
Lysis buffer just before starting has been supplemented with Lysozyms (3 mg/mL) and added to the bacterial pellet (90 µL per pellet obtained from 200 µl culture). After suspension by ambient temperature 20 µL Proteinase K (1 mg/mL) have been added and the mixture was held for 10 - 200 min by temperature 20 °C - 50 °C. After this treatment the lysates were purified by two sequential simple spin column steps: Nexttec^{™} clean column and a spin column packed with the sorbent of Example 1. In both cases the spin columns were equilibrated before use following the recommendations for the Nexttec^{™} clean column of the producer (www.nexttec.biz). After equilibration the lysat was loaded onto the column and after a short centrifugation following again the recommendation for the Nexttec^{™} clean column of the producer the eluat was collected and analysed by gel electrophoresis (1% agarose in TAE buffer).
The results of RNA preparations are shown in the following picture. Obviously, than longer the incubation time and than higher the incubation temperature, than better yield of RNA wild be obtained.

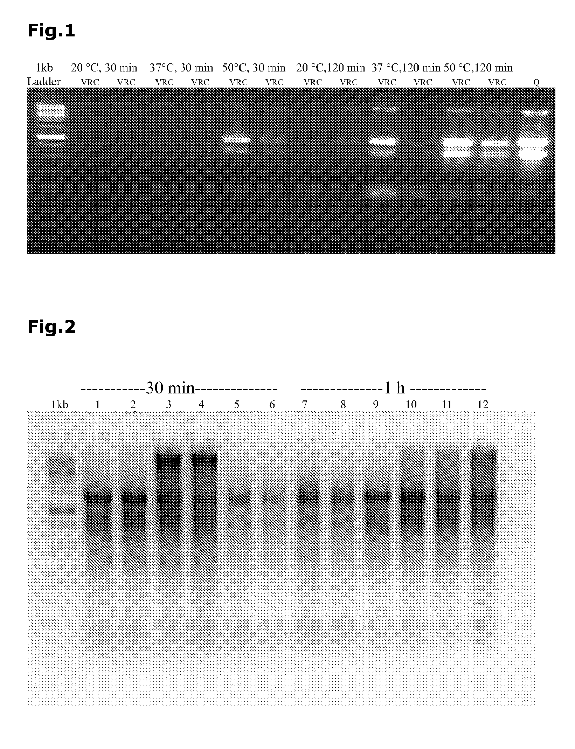

Fig. 1 shows the graph of gel electrophoresis of prepared RNA: 1 kb ladder - DNA length standard, 20 °C, 37 °C, 50 °C - incubation temperature, 30 min and 120 min - incubation time; The control lane Q shows the RNA preparation obtained with QIAGEN RNeasy.

### Example 4: RNA preparation from tissue (porcine liver) using RNase-Inhibitor ribosyl-vanadyl-complex (RVC), diethyl pyrocarbonate (DEPC) and chelating sorbent from example 1

The liver tissue was frozen overnight at -20 °C.
The comparative analysed lysis procedures differed in the composition of the lysis buffers and the incubation time. The lysis incubation was at 37 °C. All other conditions were as described under Example 3. Fig. 2 illustrates the results:

Gel electrophoresis of prepared RNA.
1 kb - DNA length standard, 0.1 % DEPC
1, 7 - lysis with VRC and 0.1 % DEPC,
2, 8 - lysis with VRC, RNasin and 0.1 % DEPC
3, 9 - lysis with VRC and Proteinase K
4,10 - lysis with VRC, RNasin and Proteinase K
5,11 - lysis with VRC, 0.1 % DEPC and Proteinase K
6,12 - lysis with VRC, 0.1 % DEPC, Proteinase K and RNasin.

As it can be deduced from Fig. 2, the longer incubation time is unfavourable, because there is no increase in RNA yield but a great release of DNA. Comparing the different variants of lysis buffer composition, the best conditions are the combination of the vanadyl-ribosyl complex with diethyl pyrocarbonat.

## Claims

1. A method for isolating RNA from RNA containing samples wherein the RNA containing sample is treated with at least one DNase and/or another enzyme like proteases or collagenases for purification of RNA in presence of a complex of ribonucleosides and the oxovanadyl ion which is capable of inhibiting RNases present in the RNA containing samples and removing the complex of ribonucleosides and the oxovanadyl ion prior to down-stream processing of the RNA of the RNA containing sample by contacting the sample of the forgoing step with a chelating agent immobilized to a support, the chelating agent having sufficient affinity to the complex of ribonucleosides and the oxovanadyl ion so that it binds to the chemical entity and the RNA containing sample is freed from the inhibitor.

2. The method of claim 1, wherein the RNA containing sample is a cell, tissue, body fluid, virus particle also in its lysed or otherwise disintegrated state.

3. The method of any of the foregoing claims wherein the chelating agent is a chemical entity comprising at least one structural moiety interacting with the oxovanadyl ion and/or the ribonucleoside-oxovanadyl complex.

4. The method according to any of the forgoing claims, wherein the chelating agent immobilized on the support is selected from the group consisting of 8-hydroxyquinoline or its derivatives, EDTA or its derivatives, phosphonic acid, its salts or derivatives, such as amides and esters, die-, tri-, tetra- or higher carboxylic acids, their salts or derivatives, such as amides, esters or nitriles.

5. A method according to any of the forgoing claims, wherein the support is an inorganic or organic polymer.

6. The method according to claims 3 or 5 wherein the support is a porous inorganic material selected from the group comprising inorganic metal oxides, such as oxides of aluminium, titanium, zirconium, silicon oxides, iron oxides, controlled pore glass (CPG), diatomaceous earth and combinations thereof.

7. A chromatographic affinity material comprising an inorganic support with immobilized chelating agents according to claims 3 and/or 4.

8. A method for manufacturing of a support according to claim 7 by contacting a reactive chemical having a moiety with affinity to a inhibitor of RNases to the support or an activated support.

9. Use of a chromatographic affinity material according to claim 7 in a method of claim 1.
